# EUROPÄISCHE PATENTSCHRIFT

(11) **EP 3 871 627 B1**
(45) Veröffentlichungstag und Bekanntmachung des Hinweises auf die Patenterteilung: **01.05.2024**
(21) Anmeldenummer: 20159760.6
(22) Anmeldetag: 27.02.2020
(51) Int. Cl.: A61B 18/12, A61B 18/14, A61B 18/00

(54) **CHIRURGISCHE EINRICHTUNG UND STEUERVERFAHREN FÜR DIESE**
SURGICAL DEVICE AND CONTROL METHOD THEREFOR
DISPOSITIF CHIRURGICAL ET SON PROCÉDÉ DE COMMANDE

(43) Veröffentlichungstag der Anmeldung: 01.09.2021
(73) Patentinhaber: Erbe Elektromedizin GmbH, 72072 Tübingen (DE)
(72) Erfinder: Dierl, Christof, 71149 Bondorf (DE)
(74) Vertreter: Rüger Abel Patentanwälte PartGmbB

(56) Entgegenhaltungen:
- EP-A1- 1 995 679
- DE-A1-102010 060 435
- US-A1- 2019 201 081

## Beschreibung

Die Erfindung betrifft eine Einrichtung zur chirurgischen Behandlung von tierischen oder menschlichen Patienten sowie ein Verfahren zum Betrieb einer Behandlungseinrichtung.

In der Elektrochirurgie werden Instrumente angewendet, die von einem speisenden Gerät mit Strom und Spannung versorgt werden, um an dem Patienten eine gewünschte Wirkung, beispielsweise eine Koagulation, einen Schnitt oder dergleichen, hervorzurufen. Das Chirurgiegerät weist dazu einen HF-Generator zur Erzeugung eines Behandlungsstroms und eine typischerweise programmgesteuerte Steuereinrichtung auf, um den Generator gemäß vorgegebener Modes zu betreiben.

Die DE 10 2009 042 428 B4 offenbart dazu ein Verfahren zur Erzeugung eines Steuerprogramms für einen solchen Chirurgiegenerator. Das Verfahren beruht auf der Darstellung der von dem Chirurgiegenerator einzunehmenden Zustände in Abhängigkeit von vorgegebenen Ereignissen in Form eines Zustandsautomaten. Der Zustandsautomat wird dann in ein Steuerprogramm übersetzt, mit dessen Hilfe das Instrument und/oder der Generator gesteuert werden.

Der Zustandsautomat legt Bedingungen fest, die, wenn sie eintreten, den Wechsel von einem vorgegebenen Zustand in einen anderen gegebenen Zustand des Systems beschreiben. Der Zustandsautomat lässt jedoch nicht den Übergang zu Zuständen zu, die in ihm selbst nicht vorkommen. Dies stellt eine Einschränkung hinsichtlich der vom Nutzer wählbaren Zustände dar. Gleiches gilt, wenn dem Nutzer an der Bedienoberfläche eines Geräts lediglich vorgegebene Modes zur Auswahl bereitgestellt werden und im Rahmen eines Modes die Effektstärken einstellbar sind, wie es in der Praxis üblich ist. Auch hier kann der Nutzer letztendlich lediglich zwischen vorgegebenen Zuständen wählen. Die vorgegebenen Zustände sind erprobt und in ihren Wirkungen dokumentiert und somit rechtlich abgesichert. Ein direkter Zugriff auf physikalische Größen des Behandlungsstroms ist damit nicht gegeben.

Weiter ist aus der EP 3 028 657 A1 ein Verfahren zum Betrieb eines elektrochirurgischen Generators bekannt, mit dem sich Algorithmen zu Steuerung eines elektrochirurgischen Generators bereitstellen lassen. Diese Algorithmen nutzen sogenannte Konfigurations-Files, die modifizierbar sind und durch einen Interpreter abgearbeitet werden. Die Steuerung des Generators beruht somit auf den Konfigurations-Files. Über ein Nutzerinterface können physikalische Parameter wie zum Beispiel Leistung, Strom, Spannung, Energie, Koagulationsparameter und dergleichen, zum Beispiel auf einer Skala von 1 bis 10 oder 1 bis 5 eingestellt werden. Die Konfigurations-Files können am Gerät oder beispielsweise auf einem cloudbasierten Update-Server bereitgestellt werden. Außerdem ist aus der EP 1 995 679 A1 eine Einrichtung zur Steuerung eines medizinischen Geräts bekannt, dass eine allgemein verfügbare Datenbasis von vorbestimmten Gerätekonfigurationen und -parametern bei medizinischen Eingriffen nutzt. Durch die allgemein verfügbare Datenbasis ist ein Zugriff auf eingriffsspezifische Daten und am Gerät einzustellende Parameterwerte möglich. Die Parameterwerte können benutzerabhängig vorbestimmt sein und vom Benutzer durch entsprechende Eingaben abgerufen werden. Dazu muss sich der Nutzer beispielsweise ein Arzt auch Sicherheits- und Dokumentationsgründen authentifizieren. Beispielseise durch einen Schlüssel, ein Passwort, einen Barcode oder eine andere geeignete Authentifizierung. Auf diese Weise kann jeder Operateur schnell und einfach auf die von ihm bevorzugten Geräteeinstellungen zugreifen.

Im Weiteren zeigen die EP 3 028657 A1**,** die WO 2018/210792 A1**,** die EP 1 337 194 A1 und die EP 1 617 776 A2 Aspekte der Steuerung von Chirurgiegeneratoren auf Basis von Algorithmen. Insbesondere aus der EP 1 616 776 B1 geht die Programmierung eines elektrochirurgischen Generators durch den Nutzer mittels Programmbefehlen hervor. Die so erzeugte Steuerungssoftware kann auf tragbaren computerlesbaren Medien abgespeichert und/oder durch Signalübertragung empfangen oder gesendet werden.

Es ist Aufgabe der Erfindung, eine Einrichtung zur chirurgischen Behandlung von tierischen oder menschlichen Patienten sowie ein Verfahren zur Steuerung einer solchen Einrichtung zu schaffen, womit einerseits die nötige Behandlungssicherheit und andererseits eine möglichst geringe Beschränkung des behandelnden Arztes hinsichtlich seiner ärztlichen Maßnahmen erreicht werden kann.

Die Einrichtung nach Anspruch 1 und das Verfahren nach Anspruch 9 erfüllen diese an sich gegensätzlichen Forderungen:

Die erfindungsgemäße Einrichtung umfasst ein Gerät zur Versorgung wenigstens eines Instruments. Das Gerät weist einen Generator auf, der eine Spannung für das Instrument sowie den zur Versorgung des Instruments dienenden elektrischen Strom bereitstellt. Der Generator ist von einer Steuereinrichtung gesteuert, die von einem Behandler auswählbare Modes zur Auswahl durch einen Behandler bereitstellt. Zu jedem Mode gehören Modedaten, die eine Anzahl physikalischer Parameter des Stroms und/oder der Spannung vorgeben. Die Steuereinrichtung ist darauf eingerichtet, den Generator entsprechend der Modedaten eines vom Behandler ausgewählten Modes zu steuern.

Weiter weist das Gerät eine Auswahleinrichtung auf, über die der Behandler manuell einen Mode auswählen kann. Zusätzlich stellt die Auswahleinrichtung für mindestens einen, vorzugsweise mehrere oder alle der auswählbaren Modes eine Möglichkeit zur Einstellung einer gewünschten Effektstärke bereit.

Zusätzlich dazu ist das Gerät über eine geeignete Datenverbindung mit einem außerhalb des Geräts angeordneten Speicher verbindbar. Dieser Speicher kann beispielsweise eine Cloud, ein Webserver oder eine andere über eine Datenverbindung erreichbare Speicherstelle sein. Die Datenverbindung ist insbesondere ein Datennetz, beispielsweise ein privates oder ein öffentliches Datennetz, das Internet oder dergleichen. In dem Speicher sind Individual-Mode-Daten speicherbar, die zu Individual-Modes gehören, die ausgewählten Behandlern individuell zugeordnet sind. Den Individual-Mode-Daten sind dazu Kennungen zugeordnet. Die jeweilige Kennung zeigt an, welche Rechte ein ausgewählter Behandler an diesen Individual-Mode-Daten hat. Rechte können beispielsweise die Befugnis sein, die Individual-Mode-Daten zu ändern und/oder die Individual-Mode-Daten zu nutzen.

Das Gerät weist eine Authentifizierungseinrichtung auf, über die Behandler individuell identifizierbar sind. Der Behandler kann nach Authentifizierung die Individual-Mode-Daten entsprechend seiner Rechte nutzen, d.h., beispielsweise diese ändern und auf ein Gerät zu übertragen, um sie dort zu nutzen. Alternativ kann ein unterprivilegierter, lediglich zur Nachnutzung berechtigter Nutzer dazu authentifiziert sein, die Individual-Mode-Daten zur Nutzung auf sein Gerät zu übertragen.

Weiter kann die Authentifizierungseinrichtung dazu eingerichtet sein, eine Nutzung der Individual-Mode-Daten zu registrieren und, falls gewünscht oder erforderlich, an eine Abrechnungseinheit zu übertragen. Die Authentifizierung ist weiter darauf eingerichtet, die Individual-Mode-Daten nach Ende der Authentifizierung zu sperren oder zu löschen.

Mit dieser Einrichtung werden sowohl die bisherigen Sicherheitsstandards eingehalten als auch zusätzlich Behandlungsoptionen eröffnet. Ein nicht authentifizierter Nutzer hat jedenfalls die üblichen Modes auswählbar zur Verfügung und kann für die ausgewählten Modes Effektstärken einstellen. Ein authentifizierter Behandler kann, wenn er eine höchste Authentifizierungsstufe hat, Individual-Mode-Daten erstellen und/oder modifizieren und hat dabei vorzugsweise direkten Zugriff auf die physikalischen Parameter der Spannung und/oder des Stroms sowie weiterer Parameter wie beispielsweise Behandlungsdauer, maximale Leistung, maximale Energie, Regelparameter, Auswertung einer Instrumentenerkennung usw. Die so erzeugten Individual-Mode-Daten sind für diesen einen Behandler, dem die Daten somit "gehören" allein nutzbar. Der Behandler kann diese Individual-Mode-Daten im Rahmen der ärztlichen Behandlungsfreiheit ohne gesonderte Zertifizierung für seine Tätigkeit nutzen. Er kann außerdem ein Datenblatt, d.h., eine Beschreibung des von ihm erzeugten Individual-Modes erzeugen oder erzeugen lassen und diese Beschreibung mit den Individual-Mode-Daten verbinden. Somit kann er den von ihm erzeugten Individual-Mode Nachnutzern zugänglich machen, die dann nach Authentifizierung das Recht erhalten, die Individual-Mode-Daten auf ihre Geräte zu laden und dort zu nutzen, nicht aber zu verändern. Zusammen mit den Individual-Mode-Daten können sie das Datenblatt, d.h., die Modebeschreibung herunterladen und diesen Individual-Mode dann im Rahmen ihrer ärztlichen Behandlungsfreiheit nutzen. Die Nutzung kann erfasst und über ein Abrechnungsmodul im Wege eines Lizenzmodells oder anderer Abrechnungsmodelle abgerechnet werden. Die Nachnutzung der Individual-Mode-Daten kann registriert und dem Inhaber der Individual-Mode-Daten zugänglich gemacht werden.

Generell ist es möglich, die Nutzung bestimmter Modes, die Dauer der Nutzung, die Anzahl der Nutzungen oder ähnliches von Standard-Modes oder auch von Individual-Modes zu erfassen und daraus entsprechende Daten zu generieren. Es kann vorgesehen werden, dass diese Daten über eine Datenverbindung an eine zentralen Server übertragen werden. Diese Daten können die Basis für ein Abrechnungsmodell sein, das die Nutzung eines Geräts oder Instruments mit dem Nutzer abrechnet. Sind die Daten anonymisiert, könne sie zur statistischen Erfassung der Nutzung der Modes dienen.

Um eine unbefugte Nachnutzung der Individual-Mode-Daten und damit einhergehende Gefahren für Personal und Patient zu verhindern, weist die Authentifizierungseinrichtung vorzugsweise eine Abmeldeoption auf, die eine Nutzung der Individual-Mode-Daten nach Abmeldung des Authentifizierten Behandlers sperrt. Die Abmeldeoption kann zum Beispiel eine auf einem Bildschirm bereitgestellte Option sein. Alternativ kann sie mit dem Ein/Aus-Schalter des Geräts verbunden sein, sodass nach dem Ausschalten des Geräts Individual-Mode-Daten nur nach Authentifizierung berechtigter Behandler verfügbar sind. Dies ist unabhängig davon, ob die Individual-Mode-Daten in dem Gerät speicherresident gehalten worden sind oder erneut von dem außerhalb des Geräts befindlichen Speicher heruntergeladen werden müssen.

Die Zuordnung der Individual-Mode-Daten zu verschiedenen Behandlern und die Rechteverwaltung dazu ermöglicht es, dass authentifizierte Behandler, unabhängig von dem physischen Gerät, an dem sie arbeiten, unabhängig von dem Operationssaal im Krankenhaus oder einer sonstigen lokalen Begebenheit, an jede Ort, zum Beispiel über das Internet, auf ihre Individual-Mode-Daten zugreifen können. Die Nutzer können die Daten auf dafür eingerichtete Geräte herunterladen, um so die von ihnen erarbeiteten Behandlungsoptionen nutzen zu können.

Zur Unterstützung der Generierung von Individual-Modes kann ein Mode-Generator vorgesehen sein, der Nutzereingaben eines authentifizierten Behandlers bezüglich physikalischer Parameter der Spannung und/oder des Stroms oder anderer Größen (Leistung, Arbeit, Crestfaktor usw.) erfasst und daraus Individual-Mode-Daten generiert. Der Mode-Generator erlaubt Zugriff auf mehrere physikalische Parameter der bereitgestellten Spannung oder des Stroms mit oder ohne fachliche Beratung von ausgebildetem Fachpersonal in einer unterstützenden Zentralstelle, bei der vorzugsweise auch der Speicher zum Vorhalten der Individual-Modes angesiedelt ist. Physikalische Parameter der Spannung und/oder des Stroms können beispielsweise ein:

die Spitzenspannung, das Tastverhältnis der Spannung und/oder des Stroms, die Einschaltdauer, die applizierte elektrische Arbeit, die maximale Leistung, eine die Stärke oder Qualität eines erzeugten Funkens kennzeichnende Größe, die minimale Leistung, die Hüllkurvenform der Spannung oder des Stroms, die Modulationsformen, die Modulationstiefe, die zeitliche Änderung eines der vorgenannten Parameter, die Änderungen eines der vorgenannten Parameter in Abhängigkeit von Messgrößen, wie z.B. Strom, Spannung, Widerstand, usw.

Durch den Mode-Generator hat der Nutzer Zugriff auf solche physikalischen Parameter des Stroms oder der Spannung, die sonst außerhalb des Zugriffs des Behandlers liegen. Damit wird die Möglichkeit geschaffen, dass Behandler (d.h. Ärzte oder auch sonstige Benutzer) aus ihrer ärztlichen Erfahrung heraus, Modes entwickeln und für sich zur Nutzung bereithalten sowie solche Modes bekannt machen und anderen Ärzten (Nachnutzers) zur Verfügung stellen.

Individualmodes können z.B. über ein Programm oder eine App auf einem externen Computer erstellt werden. Dies kann an dem Server, an einem mit dem Server verbundenen externen Computer, einem mit dem Server oder diesem Computer angeschlossenen Gerät durch einen externen Fachmann oder an dem Chirurgiegerät selbst durch den Behandler oder eine Hilfsperson erfolgen. Der externe Computer kann die Individualmode Daten dann direkt an den Server senden oder zunächst auf das Chirurgiegerät übertragen, das die Daten dann zum Server überträgt.

Beispielsweise können Individual-Modes auf vorgegebenen Modes beruhen. Beispielsweise stellt ein Gerät der Anmelderin einen Mode "preciSECT" bereit, der zum Betrieb anatomischer Pinzetten dient. Dieser Mode zeichnet sich dadurch aus, dass die maximale Spannung und der Crestfaktor über den an der Pinzette gemessenen Widerstandswert gesteuert werden. Dieser Mode kann in der Laparoskopie nicht gut angewendet werden, weil er bei versehentlicher Berührung von Metall-Trokaren zu unerwünschten Ergebnissen führt. Mit der Möglichkeit, Individual-Mode-Daten zu erzeugen, kann ein Behandler seinen eigenen Individual-Mode erstellen, der die bei laparoskopischer Anwendung störenden Effekte meidet.

Auch wünschen manche Anwender eine größere Hämostase und/oder weniger Schneideigenschaften des Instruments, z.B. eines Elektroskalpells. Dies kann der Behandler durch Anpassung von Crestfaktor und Leistungsbegrenzung erreichen, die normalerweise als Einstellgrößen nicht zugänglich sind.

Ein anderes Beispiel ist der von der Anmelderin bereitgestellte Mode EndoCutQ. Dieser Mode bietet einen Spannungsanstieg über drei Schnittpulse, um bei Polypenabtragung mit Schlingen nicht zu schnell zu schneiden. Will er diesen Mode bei der endoskopischen Submucosa-Dissektion (ESD) mit einem Nadelinstrument nutzen, ist dies für manche Anwender störend, weil nun gerade nicht initial leicht verzögert angeschnitten werden soll. Durch Generierung eines Individual-Modes auf Basis von EndoCutQ kann sich der Behandler hier selbst schnell Abhilfe schaffen.

Ein weiteres Beispiel ist der von der Anmelderin bereitgestellte Mode SwiftCOAG. Leistung und Regelspannung dieses Modes können über die Effektstufen angepasst werden. Jedoch wünschen sich manche Anwender einen schärferen Abschnitt bei geringerem Pumpenspiel und anschließend eine ausgeprägtere Hämostase. Durch Einstellung einer initial höheren Spannung und Leistung und Reduktion dieser Parameter getriggert über den am Instrument erfassten Widerstand kann das gewünschte Verhalten realisiert werden. Damit können dem Behandler solche Behandlungsoptionen eröffnet werten, wenn er dazu Individual-Mode-Daten erstellt, die er nachfolgend zu sich immer nutzen, und, falls gewünscht, auch an Kollegen lizensieren kann.

Mit der vorgeschlagenen Einrichtungen und dem vorgeschlagenen Verfahren zur Programmierung der Einrichtung wird die Möglichkeit geschaffen, das Spektrum nutzbarer Instrumente zu erhöhen, indem vorgegeben Modes im Rahmen der Individual-Mode-Erstellung an andere Instrumente angepasst werden.

Diese und weitere Vorteile der Erfindung sowie Abwandlungen und Weiterbildungen ergeben sich aus der Zeichnung, der Beschreibung und Ansprüchen. Es zeigen:
Figur 1 das erfindungsgemäße Gerät zur Speisung eines Instruments, als Funktionsblockdarstellung,
Figur 2 die zeitlichen Verläufe der Generatorspannung und des in einem beispielhaften Anwendungsfall von dem Instrument aufgenommenen Stroms als Diagramm,
Figur 3 die zeitlichen Verläufe der Spannung und des Stroms wie gemäß Figur 2, jedoch mit einer vom Nutzer vorgenommenen Modifikation,
Figur 4 die zeitlichen Verläufe der Generatorspannung bei verschiedenen Gewebewiderständen und
Figur 5 die zeitlichen Verläufe der Generatorspannung bei verschiedenen Gewebewiderständen, jedoch mit einer vom Nutzer vorgenommenen Modifikation.

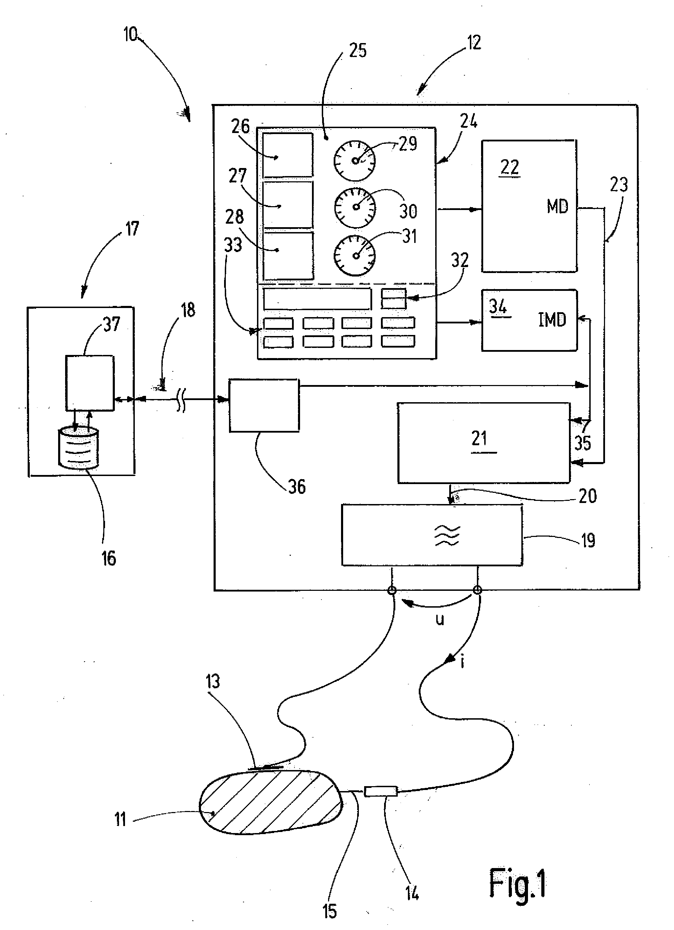

In Figur 1 ist eine Einrichtung 10 veranschaulicht, die zur Behandlung eines menschlichen oder tierischen Patienten 11 dient, der in Figur 1 lediglich symbolhaft in Gestalt eines Querschnitts eines beliebigen Körperteils veranschaulicht ist, an dem eine elektrochirurgische Behandlung vorgenommen wird. Zu der Einrichtung 10 gehören ein Gerät 12, an das eine Neutralelektrode 13 und ein Instrument 14 angeschlossen sind. Das Instrument 14 weist mindestens eine Elektrode 15 auf, über die ein Behandlungsstrom auf den Patienten 11 übertragen wird, der von der Neutralelektrode 13 aus dem Patienten wieder ausgeleitet und zu dem Gerät 12 zurückgeleitet wird. Es handelt sich bei dem Instrument 14 somit um ein monopolares Instrument. Die Erfindung ist aber nicht auf monopolare Instrumente beschränkt, sondern kann auch bei bipolaren oder multipolaren Instrumenten Anwendung finden. Auch kann das Instrument 14 ein für den offenchirurgischen Einsatz, ein für den laparoskopischen Einsatz oder ein für den endoskopischen Einsatz hergerichtetes Instrument sein.

Zu der Einrichtung 10 gehört außerdem ein externer, d.h., außerhalb des Geräts 12 ausgebildeter Speicher 16, der beispielsweise Teil eines externen Servers 17 sein kann. Der Server 17 und/oder der Speicher 16 kann über eine Datenverbindung 18 mit dem Gerät 12 zeitweilig oder dauernd verbunden sein. Die Datenverbindung 18 kann eine drahtgebundene elektrische, eine optische Datenverbindung oder eine Funkverbindung oder eine Kombination aus diesen sein. Insbesondere kann die Datenverbindung über das Internet realisiert sein.

Der externe Server 17 kann in Form einer gesonderten Hardware oder als virtuelle Maschine in einer Hardware realisiert sein, die mehrere Server aufweist und mehrere Kunden bedient. Insbesondere kann vorgesehen werden, dass der externe Server 17 mit mehreren Geräten 12 verbunden oder verbindbar ist, die an verschiedenen Orten insbesondere auch in verschiedenen Krankenhäusern oder Behandlungszentren stehen. Es ist möglich, dass alle dafür eingerichteten Geräte 12 eines Herstellers mit dem gleichen Server 17 verbunden oder verbindbar sind.

Das Gerät 12 umfasst einen Generator 19 zur Versorgung des Instruments 14 mit hochfrequenter Spannung U, sodass ein entsprechend hochfrequenter Strom i über den Patienten 11 fließen kann. Um die Spannung U und/oder den Strom i in der gewünschten Form bereitzustellen, weist der Generator 19 einen Steuereingang 20 auf, der mit einer Steuereinrichtung 21 verbunden ist. Die Steuereinrichtung 21 ist dazu eingerichtet, den Generator 19 entsprechend Mode-Daten MD zu steuern, die dazu beispielsweise in einem entsprechenden Speicher 22 bereitgehalten sind. Unter einem Speicher 22 kann auch ein Speicherbereich einer computergestützten Gerätesteuerung dienen, die alle vor und nachfolgend beschriebenen Funktionen, insbesondere auch die Funktion der Steuereinrichtung 21 realisiert.

Die Mode-Daten geben eine Anzahl physikalischer Parameter der Spannung U und/oder des Stroms i vor, wobei die Steuereinrichtung 21 darauf eingerichtet ist, den Generator 19 entsprechend dieser Vorgabe zu steuern. Die Übertragung der Mode-Daten MD aus dem zugehörigen Speicher 22 auf die Steuereinrichtung 21 ist in Figur 1 durch einen Pfeil 23 symbolisiert.

Zu dem Gerät 12 und insbesondere seiner Steuerung gehört außerdem eine Auswahleinrichtung 24, die zur Auswahl eines Modes eingerichtet ist und dazu beispielsweise ein Bedienfeld mit realen Bedienelementen oder einen Touchscreen 25 mit virtuellen Bedienelementen aufweist. Auch jede andere zur Wiedergabe von Symbolen geeigneter Einrichtung, wie beispielsweise ein Touchpad, ein Mobiltelefon oder ein anderes Ein/Ausgabegerät ist als Auswahleinrichtung 24 geeignet. Die Auswahleinrichtung 24 kann Teil des Geräts 12 oder von diesem separat ausgebildet und dann über eine Datenverbindung mit diesem verbunden sein.

Die Auswahleinrichtung 24 gestattet die Auswahl verschiedener Modes durch entsprechende Bedienelemente 26, 27, 28. Beispielsweise kann der Mode 26 ein Schneidmodus der Mode 27 ein Präzisionsschneidmodus und der Mode 28 ein Koagulationsmodus sein. Beispielsweise werden bei realen Gerät der Anmelderin drei monopolare Schneidmodi (autoCUT, highCUT, dryCUT) zwei endoskopische Schneidmodi (endoCUTQ, endoCUTI) und zwei bipolare Schneidmodi (autoCUT bipolar, highCUT bipolar) unterschieden. Zur Koagulation haben sich insgesamt 11 verschiedene Modi etabliert (softCoag, swiftCoag, TwinCoag, preciseSect, sprayCoag, forced APC, pulsed APC, precise APC, soft Coag bipolar, forced Coag bipolar, Thermosil). Jedem auswählbaren Mode 26, 27, 28 bzw. seinem Wahlschalter kann ein weiterer Wahlschalter 29, 30, 31, bzw. ein entsprechendes Eingabefeld zugeordnet sein, über das die Intensität eines Modes einstellbar ist. Die Intensität oder Effektstärke ist dabei typischerweise die einzige für den jeweils gewählte Mode 26, 27 oder 28 wähl- und vorgebbare Größe.

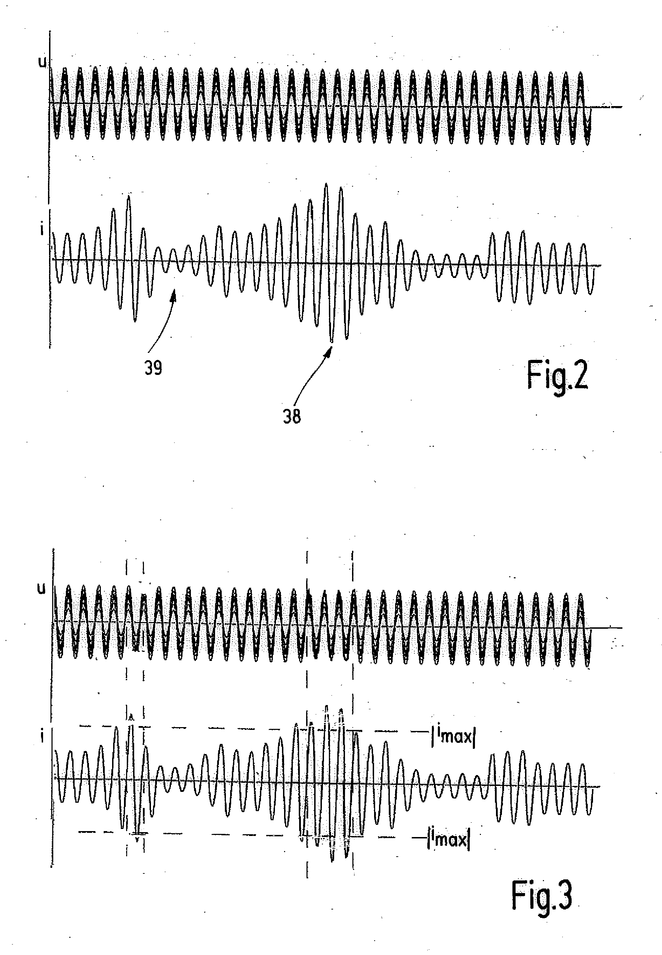

Es wird dazu auf Figur 2 verwiesen. Diese veranschaulicht oben den Verlauf der Spannung U an dem Anschluss für das Instrument 14. Lediglich beispielhaft wird dazu ein Mode mit unmodulierter HF-Spannung, zum Beispiel ein Schneidmode dargestellt. Die verschieden einstellbaren Effektstärken sind verschiedenen Spitzenspannungen der HF-Spannung zugeordnet, die der Generator 19 erzeugt. Bei niedriger Effektstärke hat die HF-Spannung U einen niedrigen Spitzenwert. Bei hoher Effektstärke hat sie einen hohen Spitzenwert. Der Spitzenwert ist beispielsweise über das Eingabemittel 29 wählbar.

Bei anderen Modes, beispielsweise dem Präzisionsschneidmodus oder dem Koagulationsmodus und auch generell, kann sich die am jeweiligen Eingabemittel 29, 30, 31 wählbare Effektstärke auch auf andere physikalische Größen beziehen. Beispiele sind: Innenwiderstand des Generators, Maximalstrom, Minimalstrom, maximale Leistung, minimale Leistung, umgesetzte elektrische Arbeit, Crestfaktor, Modulationsart, Puls/Pause-Verhältnis oder Tastverhältnis bei Ein/Aus-Modulation, Anstiegszeiten und/oder Abfallzeiten eines einzelnen HF-Spannungsimpulses, Wiederholraten von Pulen oder Pulszügen, maximale Einschaltdauern, Dauer einer erhöhten Leistung zu Aktivierungsbeginn, Höhe der aktivierten Leistung zu Aktivierungsbeginn und dergleichen mehr. Jedenfalls aber ist die Art und Weise, wie das Eingabemittel auf die gerade genannten physikalischen Größen einwirkt, um die Effektstärke zu beeinflussen, festgelegt und mit den bislang diskutierten Eingabemitteln und Wahlschaltern nicht variierbar.

Die von dem Gerät 12 im Rahmen der insoweit beschriebenen Einstellmöglichkeiten bereitgestellten Modes sind vom Gerätehersteller erprobt und zertifiziert. Zu jedem dieser Modes 26, 27, 28 und zu jedem weiteren allgemein bereitgestellten Mode existiert eine genaue Modebeschreibung, die im Speicher des Geräts 12 bereitgehalten oder von dem Server 17 herunterladbar ist. Der Behandler kann sich somit schnell und sicher über die vorhandenen Modes und deren Anwendungsweise informieren. Die Beschränkung der Betriebsarten des Generators 19 auf die in dem Speicher 22 gehaltenen Mode-Daten MD dient somit der Patientensicherheit. Sie schränkt aber die Anwendungsmöglichkeit des Geräts 10 ein.

Erfindungsgemäß ist vorgesehen, dass der Behandler an dem Gerät 12 zusätzlich Individual-Modes nutzen kann, die nicht allgemein zertifiziert und freigegeben sind. Dazu enthält die Auswahleinrichtung 24 eine Authentifizierungseinrichtung 32, über die der Nutzer ein eigenes Nutzerkonto einrichten und sich dann beispielsweise über Name und Passwort stets in sein Nutzerkonto einloggen kann. Dieses Nutzerkonto wird vorzugsweise primär in dem Server 17 angelegt. Zusätzlich oder alternativ kann es in dem Gerät 12 als Original oder als Kopie gehalten werden. Hat sich der Nutzer an dem Gerät 12 angemeldet, erhält er über weitere Bedienfelder 33 oder sonstige Eingabemittel direkten Zugriff auf physikalische Parameter der Spannung U oder des Stroms i, wie beispielsweise: maximale oder minimale Spitzenspannung, maximalen der minimalen Spitzenstrom, maximale oder minimale Leistung, maximale oder minimale verrichtete Arbeit, maximale oder minimale Einschaltdauer, Anschnittparameter (Strom, Spannung oder sonstige Größen bei Schnittbeginn), Tastverhältnisse, Anschwingzeiten, Abklingzeiten, Pausenzeiten, Regelparameter usw. Beispielsweise kann der Behandler über die Bedienfelder 33 einen vorgefertigten und bereitgestellten Mode auswählen und diesen über die Einstellung der Effektstärke hinaus variieren. Auf dieser Basis erstellt der Behandler Individual-Mode-Daten IMD, die in einem zugehörigen Speicher 34 bereitgehalten werden können. Die Bedienfelder 33 und die an diese angeschlossenen Softwaremodule bilden somit einen Individualmode-Generator.

Der Speicher 34 kann ein Speicherbereich der Gerätesteuerung oder auch ein gesonderter Speicher sein. Die Individual-Mode-Daten werden bei Aktivierung an die Steuereinrichtung 21 übertragen, was in Figur 1 durch einen Pfeil 35 symbolisiert ist. Damit kann der Behandler einen modifizierten oder gänzlich eigenen Mode erstellen und für seine Behandlungszwecke im Rahmen der ärztlichen Behandlungsfreiheit nutzen.

Weiter kann das Gerät 12 ein Kommunikationsmodul 36 aufweisen, das über die Datenverbindung 18 mit einem Kommunikationsblock 37 des Servers 18 verbunden oder verbindbar ist. Das Kommunikationsmodul 36 ist dabei darauf eingerichtet, die von dem authentifizierten Behandler erstellten Individual-Mode-Daten IMD an den Server 17 und über den Kommunikationsblock 37 an den Speicher 16 zu übertragen.

Der Server 17 dient aber, zumindest vorzugsweise, nicht lediglich der passiven Abspeicherung der von dem authentifizierten Nutzer erstellten Individual-Mode-Daten IMD. Vielmehr kann der Kommunikationsblock 37 mit Hilfe geeigneter Hilfsprogramme Unterstützung bei der Erstellung von Individual-Mode-Daten IMD erhalten. Beispielsweise können Mittel zur Überprüfung der Individual-Mode-Daten IMD vorgesehen sein, die eine thermische oder elektrische Überlastung des Instruments 14 des Generators 19 oder auch eine zu erwartende Schädigung des Patienten 11 anzeigen. Solche Mittel können einfache Komparatoren sein, die beispielsweise ein Überschreiten von Leistungsgrenzwerten am Patienten oder von Maximalspannungen oder Maximalströmen anzeigen. Es kann sich außerdem um eine künstliche Intelligenz auf Basis von Algorithmen oder auch eine trainierbare künstliche Intelligenz handeln, die anhand mehrerer von verschiedenen Behandlern erstellten Individual-Mode-Daten IMD Warnungen abgibt, wenn signifikant von sonst erstellten Individual-Mode-Daten IMD abgewichen wird. Auch kann der Kommunikationsblock 37 eine Schnittstelle für einen menschlichen Behandler enthalten, der zum Beispiel als medizinisch und technisch gebildete Person als Ansprechpartner zur Verfügung steht und die Individual-Mode-Daten IMD überprüft.

Zur Erläuterung des Betriebs auf Basis von Individual-Mode-Daten IMD wird zunächst wieder auf Figur 2 verwiesen. Es wird dabei davon ausgegangen, dass der Nutzer zunächst einen herkömmlichen zertifizierten Mode, beispielsweise den Schneidmode 26 und dabei die höchste Effektstärke, d.h., den höchsten Spannungsspitzenwert gewählt hat. Bei der Arbeit am Patienten mag sich der in Figur 2 unten dargestellte Stromverlauf ergeben. Dieser Stromverlauf ist minimal, wenn Gewebe mit hohem Widerstand stromdurchflossen ist, und maximal, wenn Gewebe mit niedrigem Gewebewiderstand stromdurchflossen ist. Zur Verdeutlichung der Erfindung sei nun angenommen, dass die sich ergebenden Spitzenströme zum Beispiel in Figur 2 bei 38 unerwünscht seien. Der Behandler könnte solche Spitzenströme vermeiden, indem er eine niedrigere Effektstärke einstellt. Diese würden aber bei Gewebe mit hohem Widerstand, wie beispielsweise bei 39, zu allzu niedrigen Strömen führen. Bei dem erfindungsgemäßen Gerät hat der Behandler nun dafür die Möglichkeit der Erstellung eines Individual-Modes, wie es in Figur 3 veranschaulicht ist. Er legt dazu beispielsweise einen Maximalbetrag für den Spitzenstrom |iₘₐₓ| fest. Er hat damit einen individuellen Mode geschaffen, bei dem bei maximaler Effektstärke der Strom dennoch einen Maximalbetrag |iₘₐₓ| nicht überschritten wird.

Diesen Individual-Mode kann der Behandler in dem Speicher 34 abspeichern und für spätere Wiederholung abrufbar machen. Er muss sich dazu an dem Gerät 12 authentifizieren. Dieser Individual-Mode ist nur für denjenigen Behandler im Rahmen seiner ärztlichen Behandlungsfreiheit bereitgehalten, der für die Nutzung dieses Individual-Modes authentifiziert ist. Ein solcher Behandler ist entweder der Ersteller des Individual-Modes oder eine Person, die die Berechtigung zur Nutzung erhalten hat.

Der Behandler kann die Individual-Mode-Daten IMD jedoch in dem Server 17 auch für Kollegen zum Download bereitstellen. Seine Individual-Mode-Daten IMD erhalten dazu eine Kennung, beispielsweise einen Namen, den der authentifizierte Behandler über die Eingabeeinrichtung 25 angibt und auf den Server 17 hoch lädt. Der authentifizierte Behandler kann dazu auch die Bedingungen der Nachnutzung, beispielsweise Lizenzbedingungen, vorgeben oder aus einer vorgegebenen Liste auswählen und mit seinem Individual-Mode verbinden.

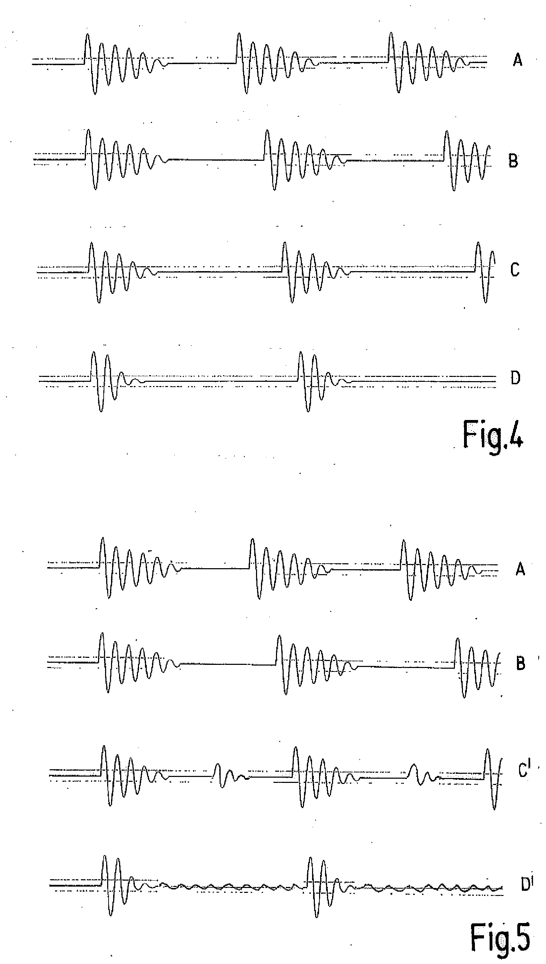

Die Erstellung von Individual-Mode-Daten IMD ist nicht auf die Begrenzung von Spannung oder Ströme oder anderen elektrischen Größen beschränkt. Es können auch qualitative Veränderungen an Modes vorgenommen oder eigene Modes erstellt werden. Dazu wird auf die Figuren 4 und 5 verwiesen. In Figur 4 ist der Präzisionsschneid-Mode 27 veranschaulicht, bei dem sich die Spannung U an erfasste Gewebewiderstände anpasst. Bei einem sehr hohen Gewebewiderstand ergibt sich der Spannungsverlauf A. Mit abnehmendem Gewebewiderstand verändert sich der Spannungsverlauf zu größeren Abständen und kürzeren Impulsen hin, bis bei sehr niedrigem Gewebewiderstand der Spannungsverlauf D erreicht ist.

Stellt der Behandler beispielsweise fest, dass er bei niedrigem und sehr niedrigem Gewebewiderstand (C und D) unerwünschtes Verhalten des Instruments 14 hat (zum Beispiel zu hohen Schneidwiderstand), kann er zur Authentifizierung den gewählten Schneid-Mode verändern. Beispielsweise kann er bei niedrigem Gewebewiderstand in die Puls-Pausen abgeschwächte Pulse einfügen, wie Figur 5 bei dem Spannungsverlauf C` veranschaulicht. Bei sehr niedrigem Gewebewiderstand kann er beispielsweise in den Puls-Pausen eine niedrige HF-Spannung applizieren, wie der Spannungsverlauf D` rein beispielhaft veranschaulicht. Auch sonstige Parameter des Spannungsverlaufs und/oder des Stromverlaufs oder anderer elektrischer Parameter können variiert werden.

Auch hier gilt wieder, dass ein so erstellter individueller Mode für andere Behandler zugänglich gemacht werden kann, indem dieser Mode als nicht zertifizierter Individual-Mode auf den Server 17 geladen und dort zur Nachnutzung nach Lizensierung bereitgestellt wird.

Die erfindungsgemäße Einrichtung zur chirurgischen Behandlung von Patienten umfasst ein Gerät 12 mit einem elektrischen Generator 19, an dem verschiedene Modes 26, 27, 28 auswählbar sind. Zusätzlich zur Auswahl vorhandener Modes 26, 27, 28 und zur Einstellung deren Effektstärke kann der authentifizierte Behandler für sich und gegebenenfalls auch ausgewählte Kollegen als Nachnutzer an dem Gerät 12 mindestens einen Individual-Mode und zugehörige Individual-Mode-Daten IMD generieren, auf Basis derer der Generator 19 außerhalb der zertifizierten Modes arbeitet. Damit werden für den Behandler Behandlungsmöglichkeiten eröffnet, die ihm sonst verschlossen bleiben. Die Generierung der Individualmodes kann bei einer Ausführungsform restriktionslos erfolgen, so dass der authentifizierte Behandler alle Einstellungen vollkommen frei innerhalb der physikalischen Grenzen des Geräts vornehmen kann. Es ist auch möglich, dass die Gerätesteuerung die Einstellmöglichkeiten limitiert, um Gefährdungen für Patienten und Personal zu begrenzen oder auszuschließen. Es ist weiter möglich, dass die Gerätesteuerung aufgrund verschiedener Authentifizierungen verschiedene Limitierungen der Einstellmöglichkeiten vorgibt.

### Bezugszeichen:

- 10: Einrichtung
- 11: Patient
- 12: Gerät
- 13: Neutralelektrode
- 14: Instrument
- 15: Elektrode
- 16: externer Speicher
- 17: externer Server
- 18: Datenverbindung
- 19: Generator
- 20: Steuereingang des Generators 20
- 21: Steuereinrichtung
- MD: Mode-Daten
- IMD: Individualmode-Daten
- 22: Speicher für Mode-Daten MD
- 23: Pfeil
- 24: Auswahleinrichtung
- 25: Touchscreen
- 26: Mode(Wahlschalter): Schneidmode
- 27: Mode(Wahlschalter): Präzisionsschneidmode
- 28: Mode(Wahlschalter): Koagulationsmode
- 29: Eingabemittel Effektstärke des Schneidmodes
- 30: Eingabemittel Effektstärke des Präzisionsschneidmodes
- 31: Eingabemittel Effektstärke des Koagulationsmodes
- 32: Authentifizierungseinrichtung
- 33: Bedienfelder, Modegenerator
- 26`, 27`: Individualmodes
- 34: Speicher für Individualmode-Daten IMD
- 35: Pfeil
- 36: Kommunikationsmodul
- 37: Kommunikationsblock
- 38: Spitzenstrom
- 39: Minimalstrom
- |iₘₐₓ|: maximaler Strombetrag

## Patentansprüche

1. Einrichtung (10) zur chirurgischen Behandlung von tierischen oder menschlichen Patienten (11),
mit einem Gerät (12) zur Versorgung wenigstens eines Instruments (14), wobei das Gerät (12) einen eine Spannung (u) erzeugenden Generator (19) zur Versorgung des Instruments (14) mit elektrischem Strom (i) und eine Steuereinrichtung (21) aufweist, die dazu eingerichtet ist, den Generator (19) entsprechend auswählbarer Modes (26, 27, 28) zu steuern, die in der Steuereinrichtung (21) gespeichert und zur Auswahl bereitgehalten sind,
wobei zu jedem auswählbaren Mode (26, 27, 28) Mode-Daten (MD) gehören, die eine Anzahl physikalischer Parameter des Stroms (i) und/oder der Spannung (u) vorgeben und wobei die Steuereinrichtung (21) darauf eingerichtet ist, den Generator (19) entsprechend der Mode-Daten (MD) eines ausgewählten Modes (26, 27, 28) zu steuern,
mit einer Auswahleinrichtung (24), die zur manuellen Auswahl eines Modes (26, 27, 28) eingerichtet ist und die zusätzlich für zumindest einige der auswählbaren Modes (26, 27, 28) zur Einstellung einer gewünschten Effektstärke eingerichtet ist,
mit einem außerhalb des Geräts (12) angeordneten Speicher (16), in dem Individualmode-Daten (IMD) speicherbar sind, die zu Individual-Modes (26', 27') gehören, die ausgewählten Behandlern individuell zugeordnet sind,
mit einer Authentifizierungseinrichtung (32), über die Behandler individuell identifizierbar sind und die dazu eingerichtet ist, die Übertragung von Individual-Mode-Daten (IMD) des authentifizierten Behandlers auf das Gerät (12) und/oder den Zugriff auf in einem Speicher (34) des Geräts gespeicherte Individual-Mode-Daten (IMD) zur Steuerung des Generators (21) freizugeben oder solche Individual-Mode-Daten (IMD) zu erstellen, und die weiter dazu eingerichtet ist, die Individualmode-Daten (IMD) zu sperren oder zu löschen, wenn die Authentifizierung endet,
**dadurch gekennzeichnet,**
**dass** zur Generierung eines Individual-Modes (26', 27') ein Mode-Generator (33) vorgesehen ist, der Nutzereingaben eines authentifizierten Behandlers bezüglich physikalischer Parameter der Spannung (u) und/oder des Stroms (i) erfasst und zugehörige Individual-Mode-Daten (IMD) generierend ausgebildet ist,
und **dass** die Individual-Mode-Daten (IMD) solche physikalischen Parameter des Stroms (i) oder der Spannung (u) betrifft, die außerhalb der im Rahmen der Auswahl der Modes und Effektstärken verfügbaren Einstellmöglichkeiten liegen.

2. Einrichtung nach Anspruch 1, **dadurch gekennzeichnet, dass** die Steuereinrichtung (21) darauf eingerichtet ist, anhand der eingestellten Effektstärke wenigstens einen der physikalischen Parameter (Spannung u, Strom i, Leistung P, Arbeit W) des gewählten Modes (26, 27, 28) zu beeinflussen.

3. Einrichtung nach einem der vorstehenden Ansprüche, **dadurch gekennzeichnet, dass** der außerhalb des Geräts (12) angeordnete Speicher (16) zu einem Server (17) gehört, der über eine Datenverbindung (18) mit dem Gerät (12) verbunden oder verbindbar ist.

4. Einrichtung nach einem der vorstehenden Ansprüche, **dadurch gekennzeichnet, dass** die Authentifizierungseinrichtung (32) zur Beendigung der Authentifizierung eine Abmeldeoption bereitstellend ausgebildet ist.

5. Einrichtung nach einem der vorstehenden Ansprüche , **dadurch gekennzeichnet, dass** die Individual-Mode-Daten (IMD) zusammen mit einer den authentifizierten Behandler identifizierenden Kennung auf den Speicher (34) übertragbar sind.

6. Einrichtung nach Anspruch 5, **dadurch gekennzeichnet, dass** die einem authentifizierten Behandler zugeordneten, in dem Speicher (34) abgelegten Individual-Mode-Daten (IMD) für einen anderen authentifizierten Behandler (Nachnutzer) zum Herunterladen freischaltbar sind.

7. Verfahren zum Betrieb einer Behandlungseinrichtung (10) :
bei dem ein Generator (19) eines Geräts (12) zur Versorgung wenigstens eines Instruments (14) eine Spannung (u) erzeugt und einen elektrischen Strom (i) an das Instrument (14) liefert, wobei der Generator (19) entsprechend auswählbarer Modes (26, 27, 28) gesteuert wird, die in einem Speicher (21) gespeichert und zur Auswahl bereitgehalten werden,
wobei zu jedem auswählbaren Mode (26, 27, 28) vorgegebene, seitens des Nutzers nicht veränderbare Mode-Daten (MD), die eine Anzahl von physikalischer Parameter der Spannung (u) und/oder des Stroms (i) festlegen, und seitens des Nutzers einstellbare Effektstärken gehören,
wobei ein Behandler mit einer Authentifizierungseinrichtung (32) individuell identifiziert wird, wodurch die Übertragung von Individual-Mode-Daten (IMD) des authentifizierten Behandlers auf das Gerät (12) und/oder der Zugriff auf in dem Speicher (34) des Geräts (12) gespeicherte Individual-Mode-Daten (IMD) zur Steuerung des Generators (19) oder die Erstellung solcher Individualmode-Daten (IMD) freigegeben wird, und wobei weiter die Individual-Mode-Daten (IMD) gesperrt oder gelöscht werden, wenn die Authentifizierung endet,
**dadurch gekennzeichnet, dass** zur Generierung der Individual-Mode-Daten (IMD) solche physikalischen Parameter des Stroms (i) oder der Spannung (u) festgelegt werden, die außerhalb der im Rahmen der Auswahl der Modes und Effektstärken verfügbaren Einstellmöglichkeiten liegen.

8. Verfahren nach Anspruch 7, **dadurch gekennzeichnet, dass** die Individual-Mode-Daten (IMD) in einem außerhalb des Geräts (12) angeordneten Speicher (16) gespeichert werden, die zu Individual-Modes (26', 27') gehören, die ausgewählten Behandlern individuell zugeordnet sind.

9. Verfahren nach Anspruch 7 oder 8, **dadurch gekennzeichnet, dass** die Individual-Mode-Daten (IMD) auf einem Server (17) gespeichert werden, der über eine Datenverbindung (18) mit dem Gerät (12) verbunden oder verbindbar ist.

10. Verfahren nach einem der Ansprüche 7 bis 9, **dadurch gekennzeichnet, dass** die Authentifizierung mittels einer Abmeldeoption beendet wird.

11. Verfahren nach einem der Ansprüche 7 bis 10, **dadurch gekennzeichnet, dass** ein Individual-Mode (26', 27') mittels eines Mode-Generators (33) erzeugt wird, der Nutzereingaben eines authentifizierten Behandlers bezüglich physikalischer Parameter der Spannung (u) und/oder des Stroms (i) erfasst und zugehörige Individual-Mode-Daten (IMD) generiert.

12. Verfahren nach einem der Ansprüche 7 bis 11, **dadurch gekennzeichnet, dass** die Individualmode-Daten (IMD) zusammen mit einer den authentifizierten Behandler identifizierenden Kennung auf den Speicher (16) übertragen werden und dass die einem authentifizierten Behandler zugeordneten, in dem Speicher (16) abgelegten Individualmode-Daten für einen anderen authentifizierten Behandler zum Herunterladen freigeschaltet werden.

## Claims

1. Device (10) for surgical treatment of animal or human patients (11),
having an apparatus (12) for supply of at least one instrument (14), wherein the apparatus (12) comprises a generator (19) creating a voltage (u) for supply of the instrument (14) with electric current (i) and a control device (21) that is configured to control the generator (19) according to selectable modes (26, 27, 28) that are stored in the control device (21) and are provided for selection,
wherein mode data (MD) are assigned to each selectable mode (26, 27, 28) that define a number of physical parameters of the current (i) and/or the voltage (u) and wherein the control device (21) is configured to control the generator (19) according to the mode data (MD) of a selectable mode (26, 27, 28),
having a selection device (24) that is configured for manual selection of a mode (26, 27, 28) and that is in addition configured for at least some of the selectable modes (26, 27, 28) for adjustment of a desired effect strength,
having a memory (16) arranged outside of the apparatus (12) in which individual mode data (IMD) can be stored that are assigned to individual modes (26', 27') that are individually assigned to selected treating persons, having an authentication device (32) by means of which treating persons can be individually identified and that can be configured to allow the transmission of individual mode data (IMD) of the authenticated treating person on the apparatus (12) and/or the access to individual mode data (IMD) stored in a memory (34) of the apparatus for control of the generator (19) or to create such individual mode data (IMD) and that is further configured to block or delete individual mode data (IMD), if the authentication terminates,
**characterized**
**in that** a mode generator (33) is provided for generation of selectable modes (26', 27') that detects user inputs of an authenticated treating person with regard to physical parameters of the voltage (u) and/or the current (i) and is configured to generate assigned individual mode data (IMD),
and **in that** the individual mode data (IMD) refer to such physical parameters of the current (i) or the voltage (u) that are beyond the available adjustment possibilities in the context of selection of the mode and effect strengths.

2. Device according to claim 1, **characterized in that** the control device (21) is configured to influence at least one of the physical parameters (voltage u, current I, power P, work W) of the selected mode (26, 27, 28) based on the adjusted effect strength.

3. Device according to any of the preceding claims, **characterized in that** the memory (16) arranged outside of the apparatus (12) is part of a server (17) that is connected or can be connected with the apparatus (12) via a data connection (18).

4. Device according to any of the preceding claims, **characterized in that** the authentication device (32) is configured to provide a check-out option for determination of the authentication.

5. Device according to any of the preceding claims, **characterized in that** the individual mode data (IMD) can be transferred together with an identifier identifying the authenticated treating person to the memory (34).

6. Device according to claim 5, **characterized in that** the individual mode data (IMD) assigned to an authenticated treating person stored in the memory (34) can be made accessible for another authenticated treating person (reuser) for a download.

7. Method for operating a treatment device (10):
during which a generator (19) of an apparatus (12) for supply of at least one instrument (14) generates a voltage (u) and supplies a current (i) to the instrument (14), wherein the generator (19) is controlled according to selectable modes (26, 27, 28) stored in a memory (34) and provided for selection,
wherein mode data (MD) that define a number of physical parameters of the voltage (u) and/or the current (i) that cannot be modified by the user and effect strengths adjustable by the user are part of each selectable mode (26, 27, 28),
wherein a treating person is identified individually by means of an authentication device (32), whereby the transmission of individual mode data (IMD) of the authenticated treating person to the apparatus (12) and/or the access to individual mode data (IMD) stored in the memory (34) of the apparatus (12) for control of the generator (19) or the creation of such individual mode data (IMD) is allowed and wherein further the individual mode data (IMD) are blocked or deleted, if the authentication terminates,
**characterized in that** such physical parameters of the current (i) or the voltage (u) are defined for generation of the individual mode data (IMD) that are beyond the available adjustment possibilities in the context of the selection of the modes and effect strengths.

8. Method according to claim 7, **characterized in that** the individual mode data (IMD) are stored in a memory (16) arranged outside the apparatus (12) that are part of individual modes (26', 27') that are assigned individually to selected treating persons.

9. Method according to claim 7 or 8, **characterized in that** the individual mode data (IMD) are stored on a server (17) that is connected or can be connected with the apparatus (12) via a data connection (18).

10. Method according to any of the claims 7 to 9, **characterized in that** the authentication is terminated by means of a check-out option.

11. Method according to any of the claims 7 to 10, **characterized in that** an individual mode (26', 27') is created by means of a mode generator (33) that detects user inputs of an authenticated treating person with reference to physical parameters of the voltage (u) and/or the current (i) and generates assigned individual mode data (IMD).

12. Method according to any of the claims 7 to 11, **characterized in that** the individual mode data (IMD) are transmitted together with an identifier identifying the authenticated treating person to a memory (16) and that the individual mode data assigned to an authenticated treating person stored in the memory (16) are released for download for another authenticated treating person.

## Revendications

1. Dispositif (10) de traitement chirurgical de patients animaux ou humains (11),
comprenant un appareil (12) destiné à l'alimentation d'au moins un instrument (14), l'appareil (12) présentant un générateur (19), produisant une tension (u) et destiné à l'alimentation de l'instrument (14) avec du courant électrique, et un dispositif de commande (21) qui est conçu pour commander le générateur (19) en fonction de modes (26, 27, 28) pouvant être sélectionnés de manière correspondante, qui sont enregistrés dans le dispositif de commande (21) et sont tenus à disposition en vue de la sélection,
sachant qu'à chaque mode (26, 27, 28) sélectionnable, sont associées des données de mode (MD) qui prédéfinissent un certain nombre de paramètres physiques du courant (i) et/ou de la tension (u), et sachant que le dispositif de commande (21) est conçu pour commander le générateur (19) en fonction des données de mode (MD) d'un mode (26, 27, 28) sélectionné,
comprenant un dispositif de sélection (24) qui est conçu pour la sélection manuelle d'un mode (26, 27, 28) et qui est conçu en plus, pour au moins quelques-uns des modes (26, 27, 28) sélectionnables, pour régler une intensité d'effet souhaitée,
comprenant une mémoire (16) qui est disposée à l'extérieur de l'appareil (12) et dans laquelle peuvent être enregistrées des données de mode individuel (IMD) qui sont associées à des modes individuels (26', 27') attribués individuellement à des personnes sélectionnées pratiquant le traitement,
comprenant un dispositif d'authentification (32) à l'aide duquel les personnes pratiquant le traitement peuvent être identifiées individuellement et qui est conçu pour autoriser la transmission à l'appareil (12) de données de mode individuel (IMD) de la personne authentifiée pratiquant le traitement et/ou l'accès à des données de mode individuel (IMD) enregistrées dans une mémoire (34) de l'appareil, en vue de la commande du générateur (21), ou pour établir de telles données de mode individuel (IMD), et qui est en outre conçu pour bloquer ou effacer les données de mode individuel (IMD) lorsque l'authentification est terminée,
**caractérisé en ce que**
pour la génération d'un mode individuel (26', 27'), il est prévu un générateur de modes (33) qui détecte des saisies utilisateur d'une personne authentifiée pratiquant le traitement, concernant des paramètres physiques de la tension (u) et/ou du courant (i), et est conçu pour générer des données de mode individuel (IMD) associées,
et **en ce que** les données de mode individuel (IMD) concernent des paramètres physiques du courant (i) ou de la tension (u) qui se situent en dehors des possibilités de réglage disponibles dans le cadre de la sélection des modes et des intensités d'effet.

2. Dispositif selon la revendication 1, **caractérisé en ce que** le dispositif de commande (21) est conçu pour influencer, à l'aide de l'intensité d'effet réglée, au moins un des paramètres physiques (tension u, courant i, puissance P, travail W) du mode (26, 27) sélectionné.

3. Dispositif selon l'une des revendications précédentes, **caractérisé en ce que** la mémoire (16) installée à l'extérieur de l'appareil (12) fait partie d'un serveur (17) qui est relié ou peut être relié à l'appareil (12) via une connexion de données (18).

4. Dispositif selon l'une des revendications précédentes, **caractérisé en ce que** le dispositif d'authentification (32) est réalisé pour fournir une option de déconnexion en vue de mettre fin à l'authentification.

5. Dispositif selon l'une des revendications précédentes, **caractérisé en ce que** les données de mode individuel (IMD) peuvent être transmises à la mémoire (34), conjointement avec un identificateur identifiant la personne authentifiée pratiquant le traitement.

6. Dispositif selon la revendication 5, **caractérisé en ce que** les données de mode individuel (IMD) associées à une personne authentifiée pratiquant le traitement et enregistrées dans la mémoire (34) peuvent être débloquées pour une autre personne authentifiée pratiquant le traitement (utilisateur ultérieur), en vue du téléchargement.

7. Procédé pour faire fonctionner un dispositif de traitement (10) :
selon lequel un générateur (19) d'un appareil (12) produit une tension (u) pour l'alimentation d'au moins un instrument (14) et fournit un courant électrique (i) à l'instrument (14), le générateur (19) étant commandé en fonction de modes (26, 27, 28) sélectionnables qui sont enregistrés dans une mémoire (21) et tenus à disposition en vue de la sélection,
sachant que sont associées à chaque mode (26, 27, 28) sélectionnable, des données de mode (MD) prédéfinies, non modifiables côté utilisateur, qui définissent un certain nombre de paramètres physiques de la tension (u) et/ou du courant (i), et des intensités d'effet pouvant être réglées côté utilisateur,
sachant qu'une personne pratiquant le traitement est identifiée individuellement à l'aide d'un dispositif d'authentification (32), ce qui a pour effet d'autoriser la transmission à l'appareil (12) de données de mode individuel (IMD) de la personne authentifiée pratiquant le traitement et/ou l'accès à des données de mode individuel (IMD) enregistrées dans la mémoire (34) de l'appareil (12), en vue de la commande du générateur (19), ou pour établir de telles données de mode individuel (IMD), et sachant qu'en outre les données de mode individuel (IMD) sont bloquées ou effacées lorsque l'authentification est terminée,
**caractérisé en ce que** pour la génération des données de mode individuel (IMD), sont définis des paramètres physiques du courant (i) ou de la tension (u), qui se situent en dehors des possibilités de réglage disponibles dans le cadre de la sélection des modes et des intensités d'effet.

8. Procédé selon la revendication 7, **caractérisé en ce que** les données de mode individuel (IMD) sont enregistrées dans une mémoire (16) installée à l'extérieur de l'appareil (12) et sont associées à des modes individuels (26', 27') qui sont attribués individuellement à des personnes sélectionnées pratiquant le traitement.

9. Procédé selon la revendication 7 ou 8, **caractérisé en ce que** les données de mode individuel (IMD) sont enregistrées sur un serveur (17) qui est relié ou peut être relié à l'appareil (12) par une connexion de données (18).

10. Procédé selon l'une des revendications 7 à 9, **caractérisé en ce qu'**il est mis fin à l'authentification à l'aide d'une option de déconnexion.

11. Procédé selon l'une des revendications 7 à 10, **caractérisé en ce qu'**un mode individuel (26', 27') est généré à l'aide d'un générateur de modes (33) qui détecte des saisies utilisateur d'une personne authentifiée pratiquant le traitement, concernant des paramètres physiques de la tension (u) et/ou du courant (i), et génère des données de mode individuel (IMD) associées.

12. Procédé selon l'une des revendications 7 à 11, **caractérisé en ce que** les données de mode individuel (IMD) sont transmises à la mémoire (16), conjointement avec un identificateur identifiant la personne authentifiée pratiquant le traitement, et **en ce que** les données de mode individuel associées à une personne authentifiée pratiquant le traitement et enregistrées dans la mémoire (16) sont débloquées pour une autre personne authentifiée pratiquant le traitement, en vue du téléchargement.
